(19) **Europäisches Patentamt / European Patent Office / Office européen des brevets**

(11) **EP 1 144 024 B1**

(12) # EUROPÄISCHE PATENTSCHRIFT

(45) Veröffentlichungstag und Bekanntmachung des Hinweises auf die Patenterteilung:
**21.02.2007 Patentblatt 2007/08**

(21) Anmeldenummer: **99964666.4**

(22) Anmeldetag: **23.12.1999**

(51) Int Cl.:
**A61M 1/36** (2006.01)

(86) Internationale Anmeldenummer:
**PCT/EP1999/010338**

(87) Internationale Veröffentlichungsnummer:
**WO 2000/038761 (06.07.2000 Gazette 2000/27)**

(54) **VORRICHTUNG ZUR BESTIMMUNG DES VERTEILUNGSVOLUMENS EINES BLUTINHALTSSTOFFES WÄHREND EINER BLUTBEHANDLUNG**

DEVICE FOR DETERMINING THE VOLUME OF DISTRIBUTION OF A BLOOD COMPONENT DURING AN EXTRACORPOREAL BLOOD TREATMENT

DISPOSITIF POUR DETERMINER LE VOLUME DE DISTRIBUTION D'UN CONSTITUANT SANGUIN PENDANT UN TRAITEMENT SANGUIN EXTRACORPOREL

(84) Benannte Vertragsstaaten:
**AT BE CH CY DE DK ES FI FR GB GR IE IT LI LU MC NL PT SE**

(30) Priorität: **24.12.1998 DE 19860330**

(43) Veröffentlichungstag der Anmeldung:
**17.10.2001 Patentblatt 2001/42**

(73) Patentinhaber: **Fresenius Medical Care Deutschland GmbH**
**61352 Bad Homburg v.d.H. (DE)**

(72) Erfinder:
 • **GOLDAU, Rainer**
 **D-97440 Werneck (DE)**
 • **GRAF, Thomas**
 **D-97424 Schweinfurt (DE)**
 • **GROSS, Malte**
 **D-97072 Würzburg (DE)**

(74) Vertreter: **Luderschmidt, Schüler & Partner Patentanwälte,**
**John-F.-Kennedy-Strasse 4**
**65189 Wiesbaden (DE)**

(56) Entgegenhaltungen:
**EP-A- 0 291 421          EP-A- 0 330 892**
**WO-A-98/55166          WO-A-99/29355**
**DE-A- 3 938 662          US-A- 4 267 040**

Bemerkungen:
•Derzeit sind die WIPO-Publikationsdaten A3 nicht verfügbar.
•Die Akte enthält technische Angaben, die nach dem Eingang der Anmeldung eingereicht wurden und die nicht in dieser Patentschrift enthalten sind.

**Beschreibung**

Die Erfindung betrifft eine Vorrichtung zur Bestimmung des Verteilungsvolumens eines Blutinhaltsstoffes im Körper eines Lebewesens während einer extrakorporalen Blutbehandlung in Verbindung mit einer Vorrichtung zur extrakorporalen Blutbehandlung.

Eine wesentliche Aufgabe der Nieren des Menschen liegt in der Abscheidung harnpflichtiger Stoffe aus dem Blut und der Regelung der Wasser- und Elektrolyt-Ausscheidung. Die Hämodialyse stellt ein Behandlungsverfahren zur Kompensation von Fehlfunktionen der Nieren bezüglich der Entfernung der harnpflichtigen Stoffe und der Einstellung der Elektrolyt-Konzentration im Blut dar.

Das Blut wird bei der Hämodialyse in einem extrakorporalen Kreislauf durch die Blutkammer eines Dialysators geleitet, die über eine semipermeable Membran von einer Dialysierflüssigkeitskammer getrennt ist. Die Dialysierflüssigkeitskammer wird von einer die Blutelektrolyte in einer bestimmten Konzentration enthaltenen Dialysierflüssigkeit durchströmt. Die Stoffkonzentration (cd) der Dialysierflüssigkeit entspricht der Konzentration des Blutes eines Gesunden. Während der Behandlung wird das Blut des Patienten und die Dialysierflüssigkeit an beiden Seiten der Membran im allgemeinen im Gegenstrom mit einer vorgegebenen Flußrate (Qb bzw. Qd) vorbeigeführt. Die harnpflichtigen Stoffe diffundieren durch die Membran von der Blutkammer in die Kammer für Dialysierflüssigkeit, während gleichzeitig im Blut und in der Dialysierflüssigkeit vorhandene Elektrolyte von der Kammer höherer Konzentration zur Kammer niedrigerer Konzentration diffundieren. Durch Anlegen eines Transmembrandrucks kann der Stoffwechsel zusätzlich beeinflußt werden.

Um das Blutbehandlungsverfahren optimieren zu können, ist die Bestimmung von Parametern der Hämodialyse während der extrakorporalen Blutbehandlung (invivo) notwendig. Von Interesse ist der Wert für die Austauschleistung des Dialysators, die durch die sogenannte "Clearance" bzw. "Dialysance D" dargestellt wird.

Als Clearance für einen bestimmten Stoff K wird dasjenige virtuelle (errechnete) Blutvolumen bezeichnet, das pro Minute unter definierten Bedingungen im Dialysator vollkommen von einem bestimmten Stoff befreit wird. Die Dialysance D ist ein weiterer Begriff zur Bestimmung der Leistungsfähigkeit eines Dialysators, bei dem die Konzentration der eliminierten Substanz in der Dialysierflüssigkeit berücksichtigt wird. Neben diesen Parametern zur Beschreibung der Leistungsfähigkeit des Dialysators sind noch andere Parameter von Bedeutung, wie die Werte des wäßrigen Anteils des Blutes, des Blutvolumens und der Bluteingangskonzentration etc..

Die meßtechnisch-mathematische Quantifizierung der Blutreinigungsverfahren und die Bestimmung der vorgenannten Parameter der Dialyse ist relativ komplex. Hinsichtlich der Berechnungsgrundlagen wird auf Sargent" J.A., Gotch. F.A.,: Principles and biophysics of dialysis, in: Replacement of Renal Function by Dialysis, C. Jacobs, C. M. Kjellstrand, K. M. Koch, J. F. Winchester (Hrsg.), Kluwer Academie Publisher, Dordrecht, 1996 verwiesen.

Die Dialysance bzw. die Clearance kann für einen gegebenen Elektrolyten, beispielsweise Natrium, bei einer Ultrafiltrationsrate von Null wie folgt bestimmt werden. Die Dialysance D ist gleich dem Verhältnis zwischen dem blutseitigen Massentransport für diesen Elektrolyten ($Q_b$ x (cbi - cbo)) und der Konzentrationsdifferenz dieses Elektrolyten zwischen dem Blut und der Dialysierflüssigkeit am jeweiligen Eingang des Dialysators (cbi - cdi).

$$D = Qb \bullet \frac{cbi - cbo}{cbi - cdi} \qquad (1)$$

Aus Gründen der Massenbilanz gilt:

$$Qb \bullet (cbi - cbo) = -Qd \bullet (cdi - cdo) \qquad (2)$$

Aus den beiden oben genannten Gleichungen (1) und (2) folgt:

$$D = -Qd \bullet \frac{cdi - cdo}{cbi - cdi} \qquad (3)$$

Dabei bedeuten in (1) bis (3):

Qb = effektiver Blutfluß
Qd = Dialysierflüssigkeitsfluß
cb = Stoffkonzentration im Blut
cd = Stoffkonzentration in der Dialysierflüssigkeit
i = Eingang des Dialysators
o = Ausgang des Dialysators

Der effektive Blutfluß ist der Fluß des Blutanteils, in dem die zu entfernenden Stoffe gelöst sind, d.h., er bezieht sich auf das (wäßrige) Lösungsvolumen für diesen Stoff. Je nach Stoff kann das der Plasmawasserfluß oder der Blutwasserfluß, d.h. der gesamte Wasseranteil im Vollblut sein. Wenn man den Vollblutfluß $Q_{vb}$ ermittelt, kann Qb über einen konstanten Faktor aus $Q_{vb}$ bestimmt werden.

Für den Fall, daß die Ultrafiltrationsrate Qf nicht gleich null ist, berechnet sich die Dialysance D wie folgt:

$$D = \left[ Qd \, \frac{cdo - cdi}{cbi - cdi} \right] \bullet \left[ 1 - \frac{Qf}{Qb} \right] + Qf \qquad (4a)$$

Der diffusive Anteil der Dialysance $D_{diff}$ berechnet sich dann wie folgt:

$$D_{diff} = \left[ Qd \, \frac{cdo - cdi}{cbi - cdi} \right] \bullet \left[ 1 - \frac{Qf}{Qb} \right] \qquad (4b)$$

Für ionische Substanzen ist für die Bluteingangskonzentration der Gibb's Donnan-Koeffizient zu berücksichtigen. Hierzu sei auf den oben zitierten Artikel von Sargent und Gotch verwiesen. Dieser Korrekturfaktor wird im folgenden der Einfachheit halber vernachlässigt.

Die DE 39 38 662 C2 (EP 0 428 927 A1) beschreibt ein Verfahren zur in-vivo-Bestimmung von Parametern der Hämodialyse, bei dem der Dialysat-Elektrolyttransfer jeweils bei zwei unterschiedlichen Dialysateingangskonzentrationen gemessen wird. Unter der Annahme, daß die Bluteingangskonzentration konstant ist, wird nach dem bekannten Verfahren die Dialysance dadurch bestimmt, daß die Differenz zwischen den Differenzen der Dialysierflüssigkeits-Ionenkonzentration an der Eingangsseite und der Ausgangsseite des Dialysators zum Zeitpunkt der ersten und zweiten Messung bestimmt wird, diese durch die Differenz der Dialysierflüssigkeits-Ionenkonzentration an der Eingangsseite zum Zeitpunkt der ersten Messung und der zweiten Messung geteilt wird und mit dem Dialysierflüssigkeitsfluß multipliziert wird.

Um bei der Hämodialyse eine Aussage über die Dialysedosis machen zu können, ist der sogenannte "Kt/V" Paramter von besonderem Interesse. Zur Berechnung dieses Parameters wird das Produkt aus der Clearance K und der Behandlungszeit t gebildet und durch das Verteilungsvolumen V des zu entfernenden Stoffes, üblicherweise Harnstoff, dividiert.

Die Behandlungszeit wird vorgegeben und ist somit bekannt. Die Clearance kann für den verwendeten Dialysatortyp Tabellen entnommen oder auch online ermittelt werden (DE 39 38 662 C2). Das Verteilungsvolumen eines Stoffes kann prinzipiell über eine normale Verdünnungsmessung bestimmt werden, bei der eine genau bemessene Markerflüssigkeit in den Patienten injiziert und deren gleichmäßige Konzentration im Blut nach einer ausreichenden Verteilungszeit gemessen wird. Dies erweist sich jedoch für ein routinemäßiges Verfahren, das beispielsweise dreimal pro Woche angewendet werden muß, als zu aufwendig.

Üblicherweise wird das Verteilungsvolumen V daher mit empirischen Schätzformeln ermittelt, in die Parameter wie die Körpergröße und das Gewicht des zu behandelnden Patienten eingehen. Der für Vermittelte Wert ist allerdings sehr ungenau.

Aus der WO 98/55166 ist ein Verfahren zur Bestimmung der Masse eines Bestandteils, z. B. Harnstoff in Blut bekannt, bei dem die Konzentration des Bestandteils in der Dialysierflüssigkeit stromab des Dialysators während der Behandlung gemessen wird. Die Masse des Bestandteils wird aus der zeitlichen Änderung der Konzentration ermittelt. Das Verteilungsvolumen soll aus der Masse des Bestandteils berechnet werden. Nachteilig ist, daß die Bestimmung des Verteilungsvolumens nicht fortlaufend, sondern nur zum Ende eines Behandlungsabschnitts erfolgt.

Der Erfindung liegt die Aufgabe zugrunde, eine Vorrichtung anzugeben, die eine schnelle und automatisierte Bestimmung des Verteilungsvolumens eines Blutinhaltsstoffes im Körper eines Lebewesens während einer extrakorporalen Blutbehandlung erlaubt.

Die Lösung dieser Aufgabe erfolgt erfindungsgemäß mit den im Patentanspruch 1 angegebenen Merkmalen.

Die Bestimmung des Verteilungsvolumens eines Blutinhaltsstoffes im Körper eines Lebewesens während einer extrakorporalen Blutbehandlung beruht auf der Veränderung der Konzentration des Blutinhaltsstoffes in der Dialysierflüssigkeit im Dialysierflüssigkeitsweg während der Blutbehandlung und der Messung der Stoffkonzentration in der Dialysierflüssigkeit stromab des

Dialysators. Die Konzentration des Blutinhaltsstoffes in der Dialyseflüssigkeit wird im Dialysierflüssigkeitsweg stromauf des Dialysators verändert. Dabei sollte die Stoffkonzentration auf physiologisch vertretbare Werte eingestellt werden.

Sofern die Änderung der Stoffkonzentration stromauf des Dialysators bekannt ist, kann auf deren Messung verzichtet werden. Andernfalls wird die Stoffkonzentration nicht nur stromab, sondern auch stromauf des Dialysators gemessen.

Die Konzentration eines Stoffes in der Dialysierflüssigkeit stromauf bzw. stromab des Dialysators wird auch als Dialysierflüssigkeitseingangs- bzw. -ausgangskonzentration cdi, cdo bezeichnet. Eine Abweichung der Dialysierflüssigkeitseingangskonzentration von der Bluteingangskonzentration cbi führt zu einer Änderung der Bluteingangskonzentration cbi am Dialysator, da sich der gemessene Stoff auf die oder von der Blutseite verschiebt. Aus der zeitlichen Änderung der Bluteingangskonzentration wird dann auf das Verteilungsvolumen dieses Stoffes im Blut geschlossen.

Zur Bestimmung des Verteilungsvolumens von Natrium im Blut wird vorzugsweise die Leitfähigkeit der Dialysierflüssigkeit als physikalische oder chemische Kenngröße gemessen. Hierzu können die bekannten Leitfähigkeitssensoren verwendet werden.

Aus dem Natriumverteilungsvolumen kann auf das Harnstoffverteilungsvolumen geschlossen werden, da das Harnstoffverteilungsvolumen im wesentlichen dem Natriumverteilungsvolumen entspricht.

Nachdem das Harnstoffverteilungsvolumen bestimmt ist, kann der sogenannte "Kt/V" Parameter berechnet werden, wobei die Clearance K entweder nach dem aus der DE 39 38 662 C2 bekannten Verfahren ermittelt oder entsprechenden Tabellen für die einzelnen Dialysatortypen entnommen werden kann.

Bei der Berechnung des Verteilungsvolumens V wird zunächst von der folgenden Massenbilanzgleichung ausgegangen:

$$\int_{t}^{t+\Delta t} Qd * cdi(t')dt' - \int_{t}^{t+\Delta t}(Qd + Qf) * cdo(t')dt' = V(t + \Delta t) * cbi(t + \Delta t) - V(t) * cbi(t)$$

(5).

Gleichung (5) gibt wieder, wie sich die Blutkonzentration cbi aufgrund der Verschiebung von Stoffen in oder aus dem Blut ändert. Teilt man die Gleichung (5) durch $\Delta t$ und betrachtet dann den Grenzwert $\Delta t \rightarrow 0$, so überführt man die integrale Massenbilanz nach Gleichung (5) in einer differentielle Massenbilanz:

$$Qd * cdi(t) - (Qd + Qf) * cdo(t) = cbi(t)\frac{dV(t)}{dt} + V(t)\frac{dcbi(t)}{dt}$$

(6)

Aufgelöst nach dcbi(t)/dt und mit dV(t)/dt = -Qf ergibt dies Gleichung (7):

$$\frac{dcbi}{dt} = \frac{Qd * cdi(t) - (Qd + Qf) * cdo(t) + Qf * cbi(t)}{V(t)}$$

(7)

Gleichung (7) stellt die Basis für die weitergehenden Überlegungen dar, wobei vorausgesetzt wird, daß der zeitliche Verlauf von cdi(t) zu einer Änderung von cbi(t) führt und die Meßzeit eine ausreichende Vermischung im Blut des Patienten bedingt.

Eine Auswertung von Gleichung (7) kann in unterschiedlichster Form vorgenommen werden. Nimmt man während der Meßphase cdi(t)=const sowie cdo(t) ≈ const und cbi(t) ≈ const an, was für den Fall eines sich während der Meßzeit nur unwesentlich ändernden Konzentrationsgradienten zwischen den beiden Flüssigkeiten gut erfüllt ist (d. h. der Zähler in (7) verändert sich nur unwesentlich), so gilt für den Zeitraum t bis t+ $\Delta$t:

$$V(t) = \frac{\left(Qd * cdi(t) - (Qd + Qf) * cdo(t) + Qf * cbi(t)\right)\Delta t}{cbi(t + \Delta t) - cbi(t)} \qquad (8)$$

Die Stoffkonzentration der Dialysierflüssigkeit stromauf des Dialysators wird sprunghaft von einem ursprünglichen Wert auf einen vorgegebenen Wert erhöht, um dann sprunghaft auf einen vorgegebenen Wert verringert zu werden, wonach wieder der ursprüngliche Wert eingestellt wird. Wenn der Wert, um den die Stoffkonzentration verringert wird, doppelt so groß ist, wie der Wert, um den die Stoffkonzentration erhöht wird und das Zeitintervall, in dem die Stoffkonzentration erhöht wird, gleich dem Zeitintervall ist, in dem die Stoffkonzentration verringert wird, liegt ein symmetrischer zeitlicher Verlauf vor, der die Auswertung vereinfacht, da durch die Verringerung der Stoffkonzentration deren Erhöhung ausgeglichen wird. Um dem Patienten nicht unnötige Mengen von beispielsweise Natrium während der Behandlung zu- oder abführen zu müssen, sollte der Ausgangswert der Stoffkonzentration in der Dialysierflüssigkeit, der während der Messung verändert wird, dem Wert im Blut entsprechen.

Für den Fall unsymmetrischer Pulse für die beiden Rechteckverläufe kann die zeitliche Änderung der Konzentration im Blut durch eine Verhältnisbildung von Integralflächen exakt berechnet werden.

Die Bestimmung des Verteilungsvolumens wird vorteilhafterweise dadurch noch weiter vereinfacht, daß während der Messung das Volumen der in den Dialysator strömenden Dialysierflüssigkeit gleich dem Volumen der aus dem Dialysator strömenden Flüssigkeit ist. Dies kann mit den bekannten Bilanziereinrichtungen erreicht werden. Es ist aber auch möglich, das Verteilungsvolumen während einer weiterlaufenden Ultrafiltration des Blutes zu ermitteln.

Das Verteilungvolumen eines Blutinhaltsstoffes im Körper eines Lebewesens kann auch dann bestimmt werden, ohne die zeitliche Änderung der Konzentration des Inhaltsstoffes im Blut explizit zu ermitteln.

Im folgenden wird ein Ausführungsbeispiel einer Hämodialysevorrichtung mit einer Vorrichtung zur Bestimmung des Harnstoffverteilungsvolumens unter Bezugnahme auf die Zeichnungen näher erläutert.

Es zeigen:

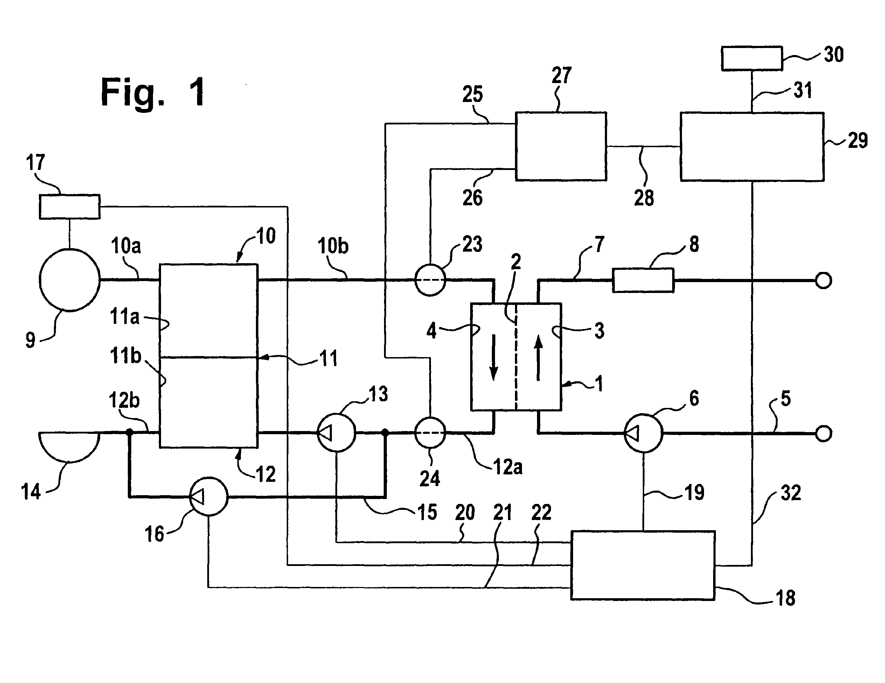

Fig. 1 eine vereinfachte schematische Darstellung der Hämodialysevorrichtung mit der Vorrichtung zur Bestimmung des Harnstoffverteilungsvolumens und

Fig. 2 den zeitlichen Verlauf der Dialysierflüssigkeitseingangs- und - ausgangskonzentration.

Die Vorrichtung zur Bestimmung des Harnstoffverteilungsvolumens kann eine separate Baugruppe bilden. Sie kann aber auch Bestandteil einer Hämodialysevorrichtung sein, zumal einige Komponenten der Vorrichtung zur Bestimmung des Harnstoffverteilungsvolumens bereits in den bekannten Dialysevorrichtungen vorhanden sind. Nachfolgend wird die Vorrichtung zur Bestimmung des Harnstoffverteilungsvolumens zusammen mit den wesentlichen Komponenten der Dialysevorrichtung beschrieben.

Die Hämodialysevorrichtung weist einen Dialysator 1 auf, der durch eine semipermeable Membran 2 in eine Blutkammer 3 und eine Dialysierflüssigkeitskammer 4 getrennt ist. Der Einlaß der Blutkammer ist mit einem Ende einer Blutzuführleitung 5 verbunden, in die eine Blutpumpe 6 geschaltet ist, während der Auslaß der Blutkammer 3 mit dem einen Ende einer Blutabführleitung 7 verbunden ist, in die eine Tropfkammer 8 geschaltet ist.

Das Dialysierflüssigkeitssystem der Hämodialysevorrichtung umfaßt eine Einrichtung 9 zur Aufbereitung der Dialysierflüssigkeit, mit der unterschiedliche Zusammensetzungen der Dialysierflüssigkeit (Elektrolytgabe) vorgegeben werden können. Die Aufbereitungseinrichtung 9 weist eine Einrichtung 17 zur Veränderung der Stoffkonzentration der Dialysierflüssigkeit, vorzugsweise der Natriumkonzentration auf. Ferner ist eine Bilanziereinrichtung vorgesehen, die zwei parallel geschaltete Bilanzkammern umfaßt, die jeweils in zwei Bilanzkammerhälften unterteilt sind. Der besseren Übersichtlichkeit halber sind hier nur die beiden Bilanzkammerhälften einer Bilanzkammer dargestellt. Über den ersten Abschnitt 10a einer Dialysierflüssigkeitszuführleitung 10 ist die Aufbereitungseinrichtung 9 mit dem Einlaß der ersten Kammerhälfte 11a der Bilanziereinrichtung 11 verbunden. Der zweite Abschnitt 10b der Dialysierflüssigkeitszuführleitung 10 verbindet den Auslaß der ersten Bilanzierkammerhälfte 11a mit dem Einlaß der Dialysierflüssigkeitskammer 4. Der Auslaß der Dialysierflüssigkeitskammer 4 ist über den ersten Abschnitt 12a einer Dialysierflüssigkeitsabführleitung 12 mit dem Einlaß der zweiten Bilanzierkammerhälfte 11b verbunden. In den ersten Abschnitt 12a der Dialysierflüssigkeitsabführleitung 12 ist eine Dialysierflüssigkeitspumpe 13 geschaltet. Der Auslaß der zweiten Bilanzierkammerhälfte 11b ist über den zweiten Abschnitt 12b der Dialysierflüssigkeitsabführleitung 12 mit einem Auslauf 14 verbunden. Stromauf der Dialysierflüssigkeitspumpe 13 zweigt von der Dialysierflüssigkeitsabführleitung 12 eine Ultrafiltratleitung 15 ab, die ebenfalls zu dem Auslauf 14 führt. In die Ultrafiltratleitung 15 ist eine Ultrafiltrationspumpe 16 geschaltet.

Die Hämodialysevorrichtung umfaßt ferner eine zentrale Steuereinheit 18, die über Steuerleitungen 19 bis 22 mit der Blutpumpe 6, der Dialysierflüssigkeitspumpe 13, der Ultrafiltrationspumpe 16 und der Einrichtung 17 zur Veränderung der Natriumkonzentration der Dialysierflüssigkeit verbunden ist.

Während der Dialysebehandlung wird die Blutkammer 3 von dem Blut des Patienten und die Dialysierflüssigkeitskammer 4 des Dialysators 1 von der Dialysierflüssigkeit durchströmt. Da die Bilanziereinrichtung 11 in den Dialysierflüssigkeitsweg geschaltet ist, kann nur so viel Dialysierflüssigkeit über die Dialysierflüssigkeitszuführleitung 10 zufließen, wie Dialysierflüssigkeit über die Dialysierflüssigkeitsabführleitung 12 abfließen kann. Mit der Ultrafiltrationspumpe 16 kann dem Patienten Flüssigkeit entzogen werden, wobei die gewünschte Ultrafiltrationsrate von der Steuereinheit vorgegeben wird.

Zur Bestimmung der Stoffkonzentration der Dialysierflüssigkeit am Eingang des Dialysators 1 (Dialysierflüssigkeitseingangskonzentration cdi) und der Stoffkonzentration der Dialysierflüssigkeit am Ausgang des Dialysators (Dialysierflüssigkeitsausgangskonzentration cdo) ist in der Zuführleitung 10 und der Abführleitung 12 stromauf bzw. stromab des Dialysators 1 jeweils eine Meßeinrichtung 23, 24 angeordnet. Die Meßeinrichtungen 23, 24 zur Bestimmung der Dialysierflüssigkeitseingangs- und -ausgangskonzentration weisen Leitfähigkeitssensoren auf, die vorzugsweise die temperaturkorrigierte Leitfähigkeit der Dialysierflüssigkeit und damit vornehmlich die Na-Konzentration messen. Anstelle von Leitfähigkeitssensoren können auch optische oder andere Sensoren, z. B. Enzymsensoren, zur Messung der Dialysierflüssigkeitseingangs- bzw. -ausgangskonzentration im Dialysierflüssigkeitsweg angeordnet sein.

Die Rechen- und Auswerteinheit 29 ist über eine Datenleitung 32 mit der Steuereinheit 18 verbunden, um die Flußraten Qb,Qd für die Blut- und Dialysierflüssigkeitspumpe 6,13 übernehmen zu können.

Die Meßeinrichtungen 23, 24 sind über Datenleitungen 25, 26 mit einer Speichereinheit 27 verbunden. Die Speichereinheit 27 empfängt die Meßwerte der Sensoren und speichert diese in zeitlicher Abfolge ab. Über eine Datenleitung 28 werden die Meßwerte einer Rechen- und Auswerteinheit 29 zugeführt, die in einem Digitalrechner (Mikroprozessor) aus den gewonnenen Daten das Harnstoffverteilungsvolumen bestimmt. Das Harnstoffverteilungsvolumen wird auf einer Ausgabeeinheit 30 angezeigt, die mit einer Datenleitung 31 mit der Auswert- und Recheneinheit 29 verbunden ist.

Zur Bestimmung des Harnstoffverteilungsvolumens arbeitet die Vorrichtung wie folgt:

Zu Beginn der Messung hält die Steuereinheit 18 die Ultrafiltrationspumpe 16 an, so daß die Ultrafiltrationsrate gleich 0 ist. Für den Blut- und Dialysierflüssigkeitsfluß gibt die Steuereinheit die Flußraten Qb und Qd vor.

Die Dialysierflüssigkeit durchströmt die Dialysierflüssigkeitskammer mit einer durch die Drehzahl der Pumpe 13 vorgegebenen Flußrate Qd und der mit der Einrichtung 17 eingestellten Dialysierflüssigkeitseingangskonzentration cdi, die von der stromauf des Dialysators angeordneten Meßeinrichtung 23 erfaßt wird. Die sich bei der Dialyse einstellende Dialysierflüssigkeitsausgangskonzentration cdo wird von der stromab des Dialysators angeordneten Meßeinrichtung 24 erfaßt.

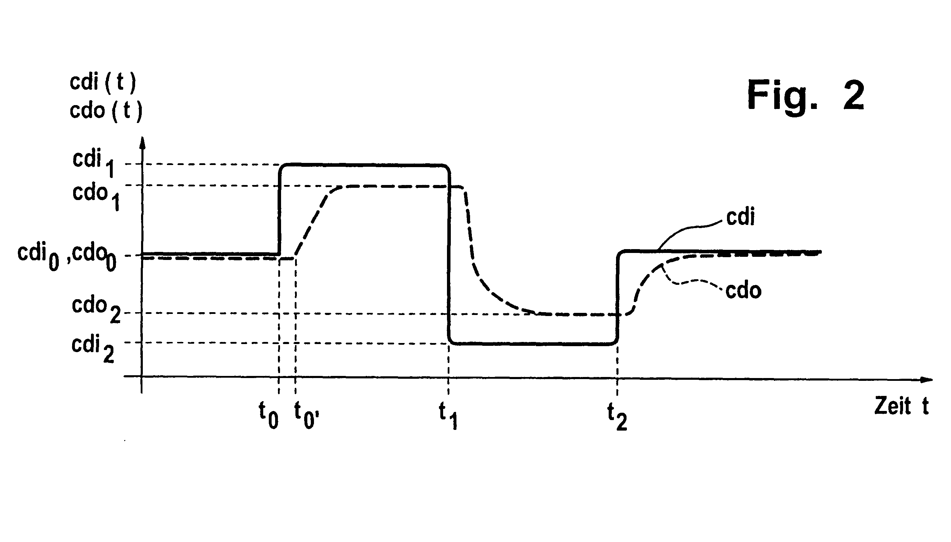

Die Einrichtung 17 stellt eine Dialysierflüssigkeitseingangskonzentration cdi(t) ein, die den in Fig. 2 gezeigten zeitlichen Verlauf hat. Ausgehend von einem für die Dialysebehandlung üblichen Wert cdi0, der dem Wert der Natriumkonzentration im Blut stromauf des Dialysators $cbi_0$ entspricht oder zumindest nahekommt, wird die Eingangskonzentration zum Zeitpunkt $t_0$ auf den Wert cdi1 erhöht. Zum Zeitpunkt $t_1$ wird die Eingangskonzentration auf den Wert $cdi_2$ verringert, um dann wieder zum Zeitpunkt $t_2$ auf den ursprünglichen Wert $cdi_0$ eingestellt zu werden.

In gestrichelten Linien zeigt Fig. 2 den zeitlichen Verlauf der sich stromab des Dialysators einstellenden Dialysierflüssigkeitsausgangskonzentration cdo(t). Für $t < t_0$ ist $cdi_0 = cdo_0$. Am Ende des Zeitintervalls $t_0 < t < t_1$ stellt sich am Dialysatorausgang ein Wert $cdo_1$ ein, während sich am Ende des Zeitintervalls $t_1 < t < t_2$ am Dialysatorausgang ein Wert $cdo_2$ einstellt. Für $t > t_2$ nimmt die Dialysierflüssigkeitsausgangskonzentration mit hinreichender Genauigkeit wieder den Wert der Dialysierflüssigkeitseingangskonzentration an. Die Dialysierflüssigkeitseingangs- und -ausgangskonzentration zeigen einen symmetrischen zeitlichen Verlauf. Der Wert, um den die Eingangskonzentration verringert wird, ist doppelt so groß wie der Wert, um den die Eingangskonzentration erhöht wird. Das Zeitintervall $t_1 - t_0$ ist gleich dem Zeitintervall $t_2 - t_1$. Die Zeitintervalle sind so bemessen, daß sich jeweils stabile Werte für cdo einstellen. Da der Verlauf symmetrisch ist, wird die durch den ersten Wechsel verursachte Verschiebung von Elektrolyten über die Membran des Dialysators wieder ausgeglichen.

Während die Dialysierflüssigkeitseingangskonzentration verändert wird, werden die Dialysierflüssigkeitseingangs- und -ausgangskonzentrationen $cdi_0$, $cdo_0$ innerhalb des Zeitintervalls $t < t_0$, $cdi_1$, $cdo_1$ innerhalb des Zeitintervalls $t_0 < t < t_1$ und $cdi_2$, $cdo_2$ innerhalb des Zeitintervalls $t_1 < t < t_2$ gemessen und in der Speichereinheit 27 abgespeichert. Dabei wird berücksichtigt, daß die Werte für cdo um eine Verzögerungszeit $t_d$ gegenüber denen von cdi zeitverschoben sind.

Da die durch den ersten Wechsel verursachte Verschiebung von Elektrolyten über die Dialysemembran im symmetrischen Fall wieder ausgeglichen wird, gilt:

$$cdi_0 = cbi_0 = cbi_2 \qquad (9)$$

wobei mit dem Index jeweils Zeitpunkte unmittelbar vor der Veränderung von cdi, als $t_0$, $t_1$ und $t_2$, bezeichnet sind.

Die zeitliche Änderung der Bluteingangskonzentration $\Delta cbi$ berechnet sich wie folgt:

$$\Delta cbi = cbi_1 - cbi_0 \qquad (10)$$

Unter der Voraussetzung, daß eine Ultrafiltrationsrate von Null (Qf=0) eingestellt ist und unter der Annahme, daß sich die Dialysance D während der Messung nicht ändert, berechnet die Rechen- und Auswerteinheit 29 $\Delta cbi$ aus den abgespeicherten Werten $cdi_0$,$cdo_0$, $cdi_1$,$cdo_1$ und $cdi_2$,$cdo_2$ sowie der eingestellten Dialysierflüssigkeitsflußrate Qd auf der Grundlage der folgenden Gleichungen:

$$D = \frac{[(cdo_0 - cdi_0) - (cdo_1 - cdi_1)]\, Qd}{(cbi_0 - cdi_0) - (cbi_1 - cdi_1)} \qquad (11)$$

$$D = \frac{[(cdo_1 - cdi_1) - (cdo_2 - cdi_2)]\, Qd}{(cbi_1 - cdi_1) - ((cbi_2 - cdi_2)} \qquad (12)$$

$$D = \frac{[(cdo_0 - cdi_0) - (cdo_2 - cdi_2)]\, Qd}{(cbi_0 - cdi_0) - (cbi_2 - cdi_2)} \qquad (13)$$

In diesen drei Gleichungen sind nur D und $\Delta cbi$ unbekannt. Die Recheneinheit ermittelt diese Parameter entweder aus zwei Gleichungen hiervon, aus Mittelwerten der jeweiligen paarweisen Kombinationen oder aus einer Variationsrechnung, die alle drei Gleichungen so gut wie möglich zu erfüllen versucht. Falls D schon bekannt sein sollte, kann dies zur weiteren Optimierung herangezogen werden.

Nachdem D und $\Delta cbi$ ermittelt sind, wird das Natriumverteilungsvolumen V in der Recheneinheit nach Gleichung (8) berechnet, wobei $\Delta cbi = cbi(t+\Delta t) - cbi(t)$ und $\Delta t = t_1 - t_2$ ist.

Hierzu liest die Rechen- und Auswerteinheit 29, die während der Messung in ihrer zeitlichen Abfolge abgespeicherten Meßwerte für die Dialysierflüssigkeitseingangs- und -ausgangskonzentration cdi (t), cdo (t) aus der Speichereinheit 27 aus. Vorzugsweise werden die Meßsignale der Leitfähigkeitssensoren abgetastet, wobei die Berechnung mit einem Digitalrechner erfolgt.

Unter der Annahme, daß das ermittelte Natriumverteilungsvolumen im wesentlichen gleich dem Harnstoffverteilungsvolumen ist, wird das Harnstoffverteilungsvolumen bestimmt und auf der Ausgabeeinheit 30 angezeigt. Aus der bekannten Clearance K und Behandlungszeit t und dem ermittelten Harnstoffverteilungsvolumen V berechnet die Rechen- und Auswerteinheit 29 den "Kt/V" Parameter, der die Dialysedosis quantifiziert. Der "Kt/V" Parameter wird auf der Ausgabeeinheit 30 ebenfalls angezeigt.

Aus den vorgenannten Gründen sollte die zeitliche Änderung der Dialysierflüssigkeitseingangskonzentration symmetrisch sein. Verwendet man jedoch unsymmetrische Pulse, kann nicht mehr angenommen werden, daß $cbi_0 = cbi_2$ ist. In diesem Fall gilt:

$$cbi_2 = cbi_0 + \Delta cbi \, (I_1 + I_2)/I_1 \tag{14}$$

$$I_1 = \int_0^{t_1} [cdi(t) - cdo(t + td)]dt \tag{15}$$

$$I_2 = -\int_{t_1}^{t_2} [cdi(t) - cdo(t + td)]dt \tag{16}$$

Die Verzögerungszeit td ist die Zeit, nach der sich die Dialysierflüssigkeitsausgangskonzentration nach der Erhöhung der Dialysierflüssigkeitseingangskonzentration erhöht. Die Verzögerungszeit td wird aus dem gemessenen zeitlichen Verlauf der Eingangs- und Ausgangskonzentrationen cdi(t) und cdo(t) berechnet. Aus Fig. 2 ist ersichtlich, daß für den Fall eines symmetrisches Verlaufes von cdi die Integralflächen $I_1$ und $I_2$ nahezu gleich sind, wodurch, wie zuvor angenommen, $cbi_2 \simeq cbi_0$ ist.

Die Berechnung des Natriumverteilungsvolumens erfolgt in der Rechen- und Auswerteinheit 29 wieder nach den Gleichungen (11) bis (13), wobei jetzt allerdings $cbi_2 = cbi_0 + \Delta cbi \, (I_1 + I_2)/I_1$ gilt. Zur Bildung der Integrale $I_1$ und $I_2$ verfügt die Rechen- und Auswerteinheit über Integratoren.

Wenn während der Messung ultrafiltriert wird, kann das Natriumverteilungsvolumen nur dann bestimmt werden, wenn die Werte $cbi_{0,1,2}$ für die Bluteingangskonzentration bekannt sind.

Hierzu kann vor der Verteilungsvolumenmessung die Bluteingangskonzentration cbi wie in der DE 39 38 662 C2 im einzelnen beschrieben, bestimmt werden, auf die ausdrücklich Bezug genommen wird. Hierbei ist es ausreichend, einen mittleren Wert für $cbi_{0,1,2}$ zu ermitteln.

Die Rechen- und Auswerteinheit 29 bestimmt nun $\Delta cbi$ auf der Grundlage der folgenden Gleichungen:

$$Ddiff = \frac{[(cdo_0 - cdi_0) - (cdo_1 - cdi_1)]Qd + [(cbi_1 - cdo_1) - (cbi_0 - cdo_0)]Qf}{\left(1 - \dfrac{Qf}{Qb}\right)[(cbi_0 - cdi_0) - (cbi_1 - cdi_1)]} \tag{17}$$

$$Ddiff = \frac{[(cdo_1 - cdi_1) - (cdo_2 - cdi_2)]Qd + [(cbi_2 - cdo_2) - (cbi_1 - cdo_1)]Qf}{\left(1 - \dfrac{Qf}{Qb}\right)[(cbi_1 - cdi_1) - (cbi_2 - cdi_2)]} \tag{18}$$

$$Ddiff = \frac{\left[(cdo_0 - cdi_0) - (cdo_2 - cdi_2)\right]Qd + \left[(cbi_2 - cdo_2) - (cbi_0 - cdo_0)\right]Qf}{\left(1 - \frac{Qf}{Qb}\right)\left[(cbi_0 - cdi_0) - (cbi_2 - cdi_2)\right]} \qquad (19)$$

$D_{diff}$ stellt den diffusiven Anteil der Dialysance dar.

Die zeitliche Änderung der Bluteingangskonzentration $\Delta$cbi wird nun ähnlich wie im Fall $Q_r=0$ aus den Gleichungen (17) bis (19) bestimmt. Dann wird das Natriumverteilungsvolumen wiederum nach Gleichung (8) berechnet. Aus dem Natriumverteilungsvolumen bestimmt die Rechen- und Auswerteinheit 29 daraufhin wieder das Harnstoffverteilungsvolumen.

Die Bestimmung des Verteilungsvolumens V eines Blutinhaltsstoffes im Körper eines Lebewesens kann in der Rechen- und Auswerteinheit 29 aber auch erfolgen, ohne die zeitliche Änderung der Konzentration des Inhaltsstoffes im Blut $\Delta$cbi explizit zu ermitteln. Hierzu stellt die Einrichtung 17 wieder den in Fig. 2 gezeigten Verlauf für die Dialysierflüssigkeitseingangskonzentration cdi(t) ein. Die Zeitpunkte "Anfang", "Ende der Hochphase" und "Ende der Niedrigphase" werden wieder mit 0, 1 bzw. 2 indiziert.

Aus der Dialysierflüssigkeitseingangs- und Ausgangskonzentration zu den Zeitpunkten i, j kann die Dialysance D wie folgt berechnet werden:

$$D_{i,j} = \left(1 - \frac{cdo_i - cdo_j}{cdi_i - cdi_j}\right)(Qd + Qf) \quad \text{Zeitindex } i \neq j \qquad (20)$$

Die obige Gleichung ist unter der Annahme hergeleitet, daß das Plasma-Natrium konstant ist. Dies ist aber aufgrund des Salztransfers nicht der Fall. Macht man diese Annahme nicht, so berechnet sich die Dialysance wie folgt:

$$D_{i,j} = \frac{\left[(cdo_i - cdi_i) - (cdo_j - cdi_j)\right]Qd + \left[(cbi_j - cdo_j) - (cbi_i - cdo_i)\right]Qf}{(cbi_i - cdi_i) - (cbi_j - cdi_j)} + Qf \qquad (21)$$

Analog lautet der diffusive Anteil der Dialysance $D_{diff}$ für eine konstante Plasma-Natrium Konzentration wie folgt:

$$D_{diff\,i,j} = \frac{\left[(cdo_i - cdi_i) - (cdo_j - cdi_j)\right]Qd + (cdo_i - cdo_j)Qf}{\left(1 - \frac{Qf}{Qb}\right)\left[(cdi_j - cdi_i)\right]} \qquad (22)$$

Läßt man die Annahme fallen, berechnet sich der diffusive Anteil der Dialysance $D_{diff}$ wie folgt:

$$D_{diff\,i,j} = \frac{\left[(cdo_i - cdi_i) - (cdo_j - cdi_j)\right]Qd + \left[(cbi_j - cdo_j) - (cbi_i - cdo_i)\right]Qf}{\left(1 - \frac{Qf}{Qb}\right)\left[(cbi_i - cdi_i) - (cbi_j - cdi_j)\right]} \qquad (23)$$

In Versuchen hat sich gezeigt, daß für das in Figur 2 gezeigte Stufenprofil die Gleichungen (20) bzw. (22) eine gute Näherung für die Gleichungen (21) bzw. (23) für i=1 und j =2 sind.

Vergleicht man nun die Werte für die Dialysance, die den Elektrolyttransfer berücksichtigen mit denen, die von einer konstanten Bluteingangskonzentration cbi herrühren, so läßt sich das Verteilungsvolumen V bestimmen.

Aus den abgespeicherten Meßwerten berechnet die Auswert- und Recheneinheit 29 zur Bestimmung des Verteilungsvolumens V zunächst nach den Gleichungen (20) und (22) für die Dialysance D und den diffusiven Anteil der Dialysance $D_{diff}$ die Werte $D_{0,1}$, $D_{1,2}$ und $D_{diff\,1,2}$. Vorzugsweise wird bei der Erfassung der Meßgrößen cdi und cdo eine Ultrafiltrationsrate Qf von Null eingestellt.

Nach der Ermittlung der obigen Werte berechnet die Auswert- und Recheneinheit dann das Verteilungsvolumen des Blutinhaltsstoffes nach der folgenden Gleichung:

$$V(t_1) = \left( \frac{D_{0,1}}{D_{1,2} - D_{0,1}} + 1 \right) \bullet \left( \frac{D_{diff\,1,2}(t_1 - t_0)(cdi_1 - cbi_0)}{cdi_1 - cdi_0} \right) \qquad (24)$$

Für ein eine konstante Ultrafiltrationsrate läßt sich das Verteilungsvolumen V für beliebeliebige Zeitpunkte t wie folgt berechnen:

$$V(t) = V(t_1) + Qf \ (t_1 - t)$$

Die Konzentration des Inhaltsstoffes im Blut $cbi_0$ (Bluteingangskonzentration) wird zuvor nach Gleichung (4a) oder (4b) bestimmt. Nachdem die Rechen- und Auswerteinheit nämlich die Dialysance bestimmt hat, ist die gesuchte Konzentration cbi die einzige Unbekannte.

Die obigen Gleichungen zeigen, daß eine Bestimmung der zeitlichen Änderung der Bluteingangskonzentration nicht explizit erforderlich ist. Es genügt die Bestimmung dieser Größe zum Zeitpunkt $t_0$.

Gleichung (24) wurde unter der Annahme hergeleitet, daß die Meßgrößen nur wenige Minuten nach einer Änderung der Dialysierflüssigkeitseingangskonzentration aufgenommen werden und sich eine mit der Zeit proportionale Verschiebung von Elektrolyten durch die Dialysatormembran einstellt. Ferner wurde angenommen, daß die Rezirkulation in der Fistel des Patientienten zu vernachlässigen ist. Zur Ausdehnung des zeitlichen Gültigkeitsbereichs und zur Berücksichtigung der Rezirkulation können noch empirisch ermittelte Korrekturfaktoren $a_i$ für $D_{0,1}$, $D_{1,2}$ und $D_{diff\,1,2}$ in Gleichung (24) berücksichtigt werden. Die Berechnung erfolgt dann nach der folgenden Gleichung:

$$V(t_1) = \left( \frac{a_1 D_{0,1}}{a_2 D_{1,2} - a_1 D_{0,1}} + 1 \right) \bullet \left( \frac{a_3 D_{diff\,1,2}(t_1 - t_0)(cdi_1 - a_4 cbi_0)}{cdi_1 - cdi_0} \right) \quad (25)$$

In Versuchen hat sich gezeigt, daß die Meßgenauigkeit insbesondere dann hoch ist, wenn der Blut- und Dialysierflüssigkeitsfluß Qb,Qd hoch ist.

## Patentansprüche

1.  Vorrichtung zur Bestimmung des Verteilungsvolumens eines Blutinhaltsstoffes im Körper eines Lebewesens während einer mit einer extrakorporalen Blutbehandlungsvorrichtung durchgeführten extrakorporalen Blutbehandlung, bei der das zu behandelnde Blut in einem extrakorporalen Kreislauf die Blutkammer (3) eines durch eine semipermeable Membran (2) in die Blutkammer und eine Dialysierflüssigkeitskammer (4) unterteilten Dialysators (1) durchströmt und Dialysierflüssigkeit in einem Dialysierflüssigkeitsweg die Dialysierflüssigkeitskammer des Dialysators durchstömt, mit
    einer Einrichtung (17) zur Veränderung der Konzentration (cdi) des Blutinhaltstoffes in der Dialysierflüssigkeit im Dialysierflüssigkeitsweg stromauf des Dialysators,
    einer Meßeinrichtung (24) zur Erfassung der Konzentration (cdo) des Stoffes in der Dialysierflüssigkeit im Dialysierflüssigkeitsweg stromab des Dialysators,
    eine Rechen- und Auswerteinheit (29), die derart ausgebildet ist, daß aus einer Änderung der Konzentration des Blutinhaltsstoffes in der Dialysierflüssigkeit im Dialysierflüssigkeitsweg stromab des Dialysators, die auf die Änderung der Konzentration des Blutinhaltsstoffes im Blut aufgrund einer Änderung der Konzentration des Stoffes in der Dialysierflüssigkeit stromauf des Dialysators zurückzuführen ist, das Verteilungsvolumen V des Blutinhaltsstoffes bestimmbar ist,

**dadurch gekennzeichnet, dass** dass die Rechen- und Auswerteinheit (29) ferner derart ausgebildet ist, dass eine von der Einrichtung (17) zur Veränderung der Stoffkonzentration verursachte Änderung der Konzentration des Blutinhaltsstoffes in der Dialysierflüssigkeit stromauf des Dialysators (1) von den Rechen- und Auswerteinheit berücksichtigt wird, wobei die Stoffkonzentration sprunghaft von einem ursprünglichen Wert auf einen vorgegebenen Wert erhöht und dann sprunghaft auf einen vorgegebenen Wert verringert wird, wonach wieder der ursprüngliche Wert eingestellt wird.

2. Vorrichtung nach Anspruch 1, **dadurch gekennzeichnet, daß** eine zweite Meßeinrichtung (23) zur Erfassung der Konzentration (cdi) des Blutinhaltstoffes in der Dialysierflüssigkeit im Dialysierflüssigkeitsweg stromauf des Dialysators (1) vorgesehen ist.

3. Vorrichtung nach Anspruch 2, **dadurch gekennzeichnet, daß** die erste und zweite Meßeinrichtung (23, 24) zur Erfassung der Stoffkonzentration einen im Dialysierflüssigkeitsweg stromab bzw. stromauf des Dialysators angeordneten Leitfähigkeitssensor, einen optischen Sensor oder einen Enzymsensor aufweisen.

4. Vorrichtung nach einem der Ansprüche 1 bis 3, **dadurch gekennzeichnet, daß** die Einrichtung (17) zur Veränderung der Stoffkonzentration derart ausgebildet ist, daß die Konzentration des Blutinhaltstoffes zu einem Zeitpunkt $t_0$ von einem vorgegebenen ersten Wert $cdi_0$ auf einen vorgegebenen zweiten Wert $cdi_1$ erhöht wird, zu einem Zeitpunkt $t_1 > t_0$ auf einen vorgegebenen dritten Wert $cdi_2$ verringert wird, und zu einem Zeitpunkt $t_2 > t_1$ auf einen vorgegebenen vierten Wert $cdi_0$ erhöht wird, der gleich dem ersten Wert ist, wobei der Wert, um den die Konzentration des Stoffes erhöht wird, halb so groß ist wie der Wert, um den die Stoffkonzentration verringert wird.

5. Vorrichtung nach Anspruch 4, **dadurch gekennzeichnet, daß** das Zeitintervall $t_1 - t_0$ gleich dem Zeitintervall $t_2 - t_1$ ist.

6. Vorrichtung nach einem der Ansprüche 1 bis 5, **dadurch gekennzeichnet, daß** eine Bilanziereinrichtung (10) vorgesehen ist, mit der die Dialysierflüssigkeit derart bilanzierbar ist, daß das Volumen der während der Messung in den Dialysator (1) strömenden Dialysierflüssigkeit gleich dem Volumen der aus dem Dialysator strömenden Dialysierflüssigkeit ist.

**Claims**

1. A device for determining the distribution volume of a blood component in the body of a living being during an extracorporeal blood treatment carried out with an extracorporeal blood treatment apparatus, wherein the blood to be treated flows in an extracorporeal circuit through the blood chamber (3) of a dialyser (1) divided by a semipermeable membrane (2) into the blood chamber and a dialysing fluid chamber (4) and dialysing fluid flows in a dialysing fluid path through the dialysing fluid chamber of the dialyser, with

a device (17) for changing the concentration (cdi) of the blood component in the dialysing fluid in the dialysing fluid path upstream of the dialyser,

a measuring device (24) for detecting the concentration (cdo) of the substance in the dialysing fluid in the dialysing fluid path downstream of the dialyser,

a computing and evaluation unit (29), which is designed in such a way that distribution volume V of the blood component can be determined from a change in the concentration of the blood component in the dialysing fluid in the dialysing fluid path downstream of the dialyser, which change can be traced back to the change in the concentration of the blood component in the blood on account of a change in the concentration of the substance in the dialysing fluid upstream of the dialyser,

**characterised in that**

the computing and evaluation unit (29) is further designed in such a way that a change in the concentration of the blood component in the dialysing fluid upstream of the dialyser (1) caused by the device (17) for changing the substance concentration is taken into account by the computing and evaluation unit,

wherein the substance concentration is increased abruptly from an original value to a preset value and is then reduced abruptly to a preset value, after which the original value is set again.

2. The device according to claim 1, **characterised in that** a second measuring device (23) is provided for detecting the concentration (cdi) of the blood component in the dialysing fluid in the dialysing fluid path upstream of the dialyser (1).

3. The device according to claim 2, **characterised in that** the first and second measuring device (23, 24) for detecting the substance concentration have a conductivity sensor, an optical sensor or an enzyme sensor arranged in the dialysing fluid path downstream or upstream of the dialyser.

4. The device according to any one of claims 1 to 3, **characterised in that** the device (17) for changing the substance concentration is designed in such a way that the concentration of the blood component is increased at a time to from a preset first value $cdi_0$ to a preset second value $cdi_1$, is reduced at a time $t_1 > t_0$ to a preset third value $cdi_2$, and at a time $t_2 > t_1$ is increased to a preset fourth value $cdi_0$ which is equal to the first value, wherein the value by which the concentration of the substance is increased its half as large as the value by which the substance concentration is reduced.

5. The device according to claim 4, **characterised in that** the time interval $t_1 - t_0$ is equal to the time interval $t_2 - t_1$.

6. The device according to any one of claims 1 to 5, **characterised in that** a balancing device (10) is provided, with which the dialysing fluid can be balanced in such a way that the volume of the dialysing fluid flowing into the dialyser (1) during the measurement is equal to the volume of the dialysing fluid flowing out of the dialyser.

## Revendications

1. Dispositif pour déterminer le volume de répartition d'un composant sanguin dans le corps d'un être vivant pendant un traitement extracorporel du sang effectué avec un dispositif extracorporel de traitement du sang,
dans lequel le sang à traiter traverse, dans un circuit extracorporel, le compartiment à sang (3) d'un dialyseur (1) comprenant le compartiment à sang et un compartiment à dialysat (4) séparés par une membrane semi-perméable (2) et dans lequel du dialysat traverse le compartiment à dialysat du dialyseur sur le parcours du dialysat, avec
un dispositif (17) destiné à modifier la concentration (cdi) du composant sanguin dans le dialysat sur le parcours du dialysat en amont du dialyseur,
un dispositif de mesure (24) destiné à détecter la concentration (cdo) du composant dans le dialysat sur le parcours du dialysat en aval du dialyseur,
une unité de calcul et d'analyse (29) qui est réalisée de telle manière que le volume de distribution V du composant sanguin peut être déterminé à partir d'une modification de la concentration du composant sanguin dans le dialysat sur le parcours du dialysat en aval du dialyseur, qui est due à la modification de concentration du composant sanguin dans le sang, qui résulte elle-même d'une modification de la concentration du composant dans le dialysat en amont du dialyseur,
**caractérisé en ce que**
l'unité de calcul et d'analyse (29) est en outre réalisée de telle manière qu'une modification de la concentration du composant sanguin dans le dialysat en amont du dialyseur (1) causée par le dispositif (17) destiné à modifier la concentration du composant est prise en compte par l'unité de calcul et d'analyse,
la concentration du composant étant subitement augmentée d'une valeur initiale à une valeur prescrite puis subitement réduite à une valeur prescrite, la valeur initiale étant à nouveau réglée.

2. Dispositif selon la revendication 1, **caractérisé en ce qu'**un deuxième dispositif de mesure (23) destiné à détecter la concentration (cdi) du composant sanguin dans le dialysat sur le parcours du dialysat est prévu en amont du dialyseur (1).

3. Dispositif selon la revendication 2, **caractérisé en ce que** le premier et le deuxième dispositif de mesure (23, 24) destinés à détecter la concentration du composant sur le parcours du dialysat présentent un capteur de conductivité disposé en aval et/ou en amont du dialyseur, un capteur optique ou un capteur d'enzymes.

4. Dispositif selon l'une quelconque des revendications 1 à 3, **caractérisé en ce que** le dispositif (17) destiné à modifier la concentration du composant est réalisé de telle manière que la concentration du composant sanguin est augmentée à un moment $t_0$ d'une première valeur $cdi_0$ prescrite à une deuxième valeur $cdi_1$ prescrite, réduite à un moment $t_1 > t_0$ à une troisième valeur $cdi_2$ prescrite, et augmentée à un moment $t_2 > t_1$ à une quatrième valeur $cdi_0$ prescrite qui est égale à la première valeur, la valeur de laquelle la concentration du composant est augmentée est égale à la moitié de la valeur de laquelle la concentration du composant est réduite.

5. Dispositif selon la revendication 4, **caractérisé en ce que** l'intervalle de temps $t_1 - t_0$ est égal à l'intervalle de temps $t_2 - t_1$.

6. Dispositif selon l'une quelconque des revendications 1 à 5, **caractérisé en ce qu'**est prévu un dispositif de bilan (10) permettant d'effectuer un bilan du dialysat de telle manière que le volume du dialysat qui afflue dans le dialyseur (1) pendant la mesure est égal au volume du dialysat qui afflue depuis le dialyseur.

# Fig. 1

EP 1 144 024 B1

Fig. 2

cdi ( t )
cdo ( t )

$cdi_1$
$cdo_1$
$cdi_0, cdo_0$
$cdo_2$
$cdi_2$

cdi

cdo

$t_0$ $t_{0'}$    $t_1$    $t_2$    Zeit t

EP 1 144 024 B1